# EUROPEAN PATENT APPLICATION

(11) **EP 2 311 435 A1**
(43) Date of publication of application: **20.04.2011**
(21) Application number: 09172391.6
(22) Date of filing: 07.10.2009
(51) Int. Cl.: A61K 9/14, A61K 9/16, A61K 9/20, A61K 47/34, A61K 31/64

(54) **Pharmaceutical composition comprising poorly soluble active ingredient and hyperbranched polymer**

(71) Applicant: LEK Pharmaceuticals d.d., 1526 Ljubljana (SI)
(72) Inventor: The designation of the inventor has not yet been filed

(57) **Abstract**

The present invention belongs to the field of pharmaceutical industry and relates to a pharmaceutical composition comprising at least one hyperbranched polymer and at least one pharmaceutically active ingredient, wherein the polymer and the pharmaceutically active ingredient are present in a specific weight ratio, and to a process for the preparation of said pharmaceutical composition.

The present invention is also directed to a powder or granulate comprising at least one hyperbranched polymer and at least one pharmaceutically active ingredient in a specific weight ratio, to a process for the preparation of said powder or granulate, and to a pharmaceutical dosage form comprising the pharmaceutical composition, powder or granulate.

Furthermore, the present invention relates to a process for preparing a solid dispersion of a hyperbranched polymer and at least one API.

Moreover, it relates to the use of a crystalline carrier for the preparation of a mixture of hyperbranched polymer and a pharmaceutically active ingredient, as well as to the use of dendrimeric polyesteramide hyperbranched polymers for the preparation of a pharmaceutical composition, to the use of a hydrophilic or hydrophobic carrier for the preparation of said composition comprising a specific group of API, and to the use of a specific API and at least one hyperbranched polymer for the preparation of a pharmaceutical composition.

## Description

### Field of the invention

The present invention belongs to the field of pharmaceutical industry and relates to a pharmaceutical composition comprising at least one hyperbranched polymer and at least one pharmaceutically active ingredient exhibiting poor solubility in aqueous solvents, wherein the hyperbranched polymer and the pharmaceutically active ingredient (API) are present in a specific weight ratio, and to a process for the preparation of said pharmaceutical composition. The present invention is also directed to a powder or granulate comprising at least one hyperbranched polymer and at least one pharmaceutically active ingredient exhibiting poor solubility in aqueous solvents, wherein the polymer and the API are present in a specific weight ratio, to a process for the preparation of said powder or granulate, and to a pharmaceutical dosage form comprising the composition, powder or granulate. The present invention further relates to a process for preparing solid dispersion of a hyperbranched polymer and at least one API, as well as to the use of a crystalline carrier for the preparation of a mixture of a hyperbranched polymer and a pharmaceutically active ingredient. Moreover, the present invention is also directed to the use of dendrimeric polyesteramide hyperbranched polymers for the preparation of a pharmaceutical composition, to the use of a hydrophilic carrier for the preparation of said composition comprising a specific group of API, and to the use of a specific API and at least one hyperbranched polymer for the preparation of a pharmaceutical composition.

### Description of the background art

Pharmaceutically active ingredients are normally not administered to patients for prophylaxis or treatment just alone, but instead the pharmaceutically active ingredients are usually formulated in pharmaceutical compositions such as powders or tablets, such as coated or uncoated mono- or multilayered tablets. Depending on the respective route of administration, pharmaceutical compositions can be specifically designed to provide the desired dissolution profile. In order to ensure that the pharmaceutically active ingredient exhibits the desired pharmacological activity it must reach a sufficient concentration at the site of action. This means that the pharmaceutically active ingredient has to exhibit a certain minimum solubility, which in turn often requires improving the solubility of the respective pharmaceutically active ingredient.

In this respect, many solubilization techniques such as the addition of cosolvent, micellar solubiilzation through surfactants, the use of cyclodextrine, pH modification, solvent recrystallization, spray drying and the like are available in order to solubiilze insoluble or poorly soluble drugs in aqueous or other desired solvents.

Furthermore, US 2009/0041813 A1 discloses compositions comprising at least one active substance which is sparingly soluble in water and at least one hyperbranched polymer comprising nitrogen atom.

The patent application W02004/072153 discloses multifunctional dendrimeric and hyperbranched polymers used as efficient drug and gene delivery systems.

However, despite the above-described compositions there is still a need for an improved pharmaceutical composition comprising pharmaceutically active ingredients, in particular with regard to the solubility of the pharmaceutically active ingredient, and for an improved process for preparing such a pharmaceutical composition.

### Summary of the invention

The present invention provides the following aspects, subject-matters and preferred embodiments, which respectively taken alone or in combination, contribute to solving the object of the present invention:
(1) A pharmaceutical composition comprising:
   a) at least one hyperbranched polymer; and
   a) at least one pharmaceutically active ingredient exhibiting solubility in an aqueous media at pH 6.8 and 37° C of less than 1 mg/ml,
   wherein the weight ratio of the at least one hyperbranched polymer to the at least one active pharmaceutical ingredient is 99:1 w/w to 11:1 w/w or wherein the weight ratio can even be 99:1 w/w to 4:1 w/w if the active pharmaceutical ingredient is selected from the group consisting of phosphodiesterase inhibitors, preferably phosphodiesterase III inhibitors or phosphodiesterase V inhibitors; non-thiazide sulphonamides; sulfonylurea derivatives; and hydroxy-1,4-naphthoquinone derivatives; or wherein the weight ratio 99:1 w/w to 4:1 w/w if a dendrimeric polyesteramide, preferably a dendrimeric polyesteramide having tertiary amine end groups or having hydroxyl end groups is used as the hyperbranched polymer. Preferably, the hyperbranched polymer and the pharmaceutically active ingredient form a solid dispersion.

A preferred phosphodiesterase III inhibitor is pimobendan, a preferred phosphodiesterase V inhibitor is tadalfil; a preferred non-thiazide sulphonamide is indapamide; a preferred sulfonylurea derivative is glimepiride; and a preferred hydroxy-1,4-naphthoquinone derivative is atovaquone. In a preferred embodiment, a dendrimeric polyesteramide having tertiary amine end groups is used in order to provide the best solubility of the API. In another embodiment of the invention, a dendrimeric polyesteramide having hydroxyl end groups is used to provide the lowest hygroscopicity of the pharmaceutical composition, powder or granulate.

Preferably, the weight ratio of hyperbranched polymer/active pharmaceutical according to the invention is 99:1 to 11:1. In a preferred embodiment, the weight ratio is 99:1 to 14:1, further preferred, the weight ratio is 99:1 to 17:1, even further preferred, the weight ratio is 99:1 to 18:1. In another preferred embodiment, the weight ratio is 80:1 to 11:1, further preferred, the weight ratio is 55:1 to 11:1, even further preferred, the weight ratio is 24:1 to 11:1. In a further embodiment, the weight ratio is 80:1 to 14:1, further preferred, the weight ratio is 55:1 to 17:1, even further preferred, the weight ratio is 24:1 to 18:1. Preferably, for example when the active pharmaceutical ingredient is glimepiride or indapamide, the weight ratio of hyperbranched polymer and said active pharmaceutical ingredient is 98:2 w/w to 95:5 w/w.
(2) The pharmaceutical composition according to item (1), wherein the mixture of hyperbranched polymer and the pharmaceutically active ingredient further comprises at least one water-dispersible or water-soluble pharmaceutically acceptable carrier. Preferably, the hyperbranched polymer and the at least one active pharmaceutical ingredient form a solid dispersion or a solid solution. Preferably, a solid dispersion is formed between the hyperbranched polymer and the at least one pharmaceutically active ingredient. Further preferred, the solid dispersion or solid solution is obtained by using known techniques (such as high shear mixing, spray drying or fluid bed granulation). Within the meaning of the present invention, a solid dispersion denotes a dispersion of one or more compound(s) in an inert carrier at solid state. The term "solid solution" denotes a solid-state solution of one or more solutes in a solvent, wherein the mixture remains in a single homogenous phase.
(3) The pharmaceutical composition according to item (1) or (2), wherein the at least one hyperbranched polymer comprises hydroxyl groups, ester groups, amido groups and/or carboxyl groups, preferably the at least one hyperbranched polymer comprises ester or amido groups, preferably, the hyperbranched polymer is a polyesteramide if the weight ratio of hyperbranched polymer and said active pharmaceutical ingredient is 11:1 or higher.
(4) The pharmaceutical composition according to items (2)-(3), wherein the water-dispersible or water-soluble carrier is selected from the group consisting of modified or unmodified carbohydrates, preferably monomeric, oligomeric or polymeric carbohydrates, preferably modified or unmodified monomeric, oligomeric or polymeric monosaccharides, or straight or branched oligosaccharides or polysaccharides; wax; gum; organic or inorganic acids or bases, or a salt thereof; surfactants; synthetic polymers; modified or unmodified silica; mineral pharmaceutical excipients or a combination thereof; preferably the carrier is crystalline.
(5) The pharmaceutical composition according to item (4), wherein the organic acid is amino acid or citric acid.
(6) The pharmaceutical composition according to any of the preceding items, wherein the carrier is sucrose, maltose, lactose, glucose, mannose, mannitol, sorbitol, xylitol, erythritol, lactitol, maltitol, a starch or modified starch, such as pregelatinized starch, corn starch, potato starch, or maize starch; an alginate, gelatin, carrageenan, dextran, maltodextran, dextrates, dextrin, polydextrose, or tragacanth; acacia, guar gum, xanthan gum; cellulose such as carboxymethylcellulose, methylcellulose, sodium carboxymethyl cellulose, hydroxypropylcellulose, hydroxypropylmethylcellulose, methyl cellulose, ethyl cellulose, hydroxyethyl cellulose, powdered cellulose, or microcrystalline cellulose; polyacrylic acid, modified or unmodified alginate or chitosan, gelatin, pectin, polyethylene, glycolpolyethylene glycol, polyvinylpyrrolidone, polyvinylalcohol, polyoxyethylene copolymers, polyoxypropylene copolymers, or polyethyleneoxide; arginine, meglumine, lysine, monoethanolamine, diethanolamine, triethanolamine, propanolamine, dipropanolamine, thiamine, sodium salicylate, or a mixture thereof.
(7) The pharmaceutical composition according to item (6), wherein the carrier is selected from the group consisting of lactose, sucrose, maltose, mannitol, sorbitol, xylitol, erythritol, lactitol, maltitol, starch and cellulose, preferably lactose, maltose, mannitol, sorbitol, starch and cellulose, more preferably lactose and cellulose, yet more preferably the carrier is lactose.
(8) Powder or granulate comprising
   a) at least one hyperbranched polymer,
   b) at least one pharmaceutically active ingredient exhibiting solubility in an aqueous media at pH 6.8 and 37° C of less than 1 mg/ml,
      wherein the weight ratio of the at least one hyperbranched polymer to the at least one active pharmaceutical ingredient is 99:1 w/w to 4:1 w/w, and
   c) a hydrophilic or hydrophobic carrier, preferably a hydrophilic carrier, more preferably the carrier is crystalline.
(9) Powder or granulate according to item (8), wherein the hyperbranched polymer and the at least one pharmaceutically active ingredient form a solid dispersion or a solid solution, preferably the solid dispersion or solid solution and the pharmaceutically active ingredient is combined with the solid or dissolved carrier. Preferably, the carrier is a solid carrier. The carrier may be combined with the solid dispersion or solid solution by using mixing, high shear mixing, spray drying, fluid bed granulation or freeze drying. Preferably the solution of step b) is sprayed onto the carrier particles.
   Preferably, a solid dispersion is formed between the hyperbranched polymer and the at least one pharmaceutically active ingredient.
(10) The pharmaceutical composition according to any one of the preceding items, wherein the amount of carrier included in the composition is from 10 % to 90 %, preferably from 30 % to 60 %, more preferably from 50 % to 80 %, yet more preferably from 70 % to 80 % based on the weight of the pharmaceutical composition and powder or granulate, respectively.
(11) The pharmaceutical composition, powder or granulate according to any one of the preceding items, wherein the at least one pharmaceutically active ingredient is selected from the group consisting of glimepiride, gliclazide, glipizide, glibenclamide, pimobendan, tadalafil, atovaquone and indapamide, preferably glimepiride, pimobendan, tadalafil, atovaquone and indapamide, more preferably the pharmaceutically active ingredient is glimepiride.
(12) The pharmaceutical composition, powder or granulate according to any of the preceding items, further comprising another pharmaceutically active ingredient. Preferably said additional pharmaceutically active ingredient is selected from the group consisting of the thiazolidinone class, biguanide class, statin class, fibrate class, thiazide class, and acarbose, miglitol, voglibose, orlistat, sibutramine, rimonabant, exenatide and pramlintide; preferably said additional pharmaceutically acive ingredient is selected from the biguanide class, thiazolidinone class and thiazide class; more preferably it is selected from the thiazolidinone class. Pharmaceutically active ingredients from the thiazolidinone class are for example pioglitazone, rivoglitazone, rosiglitazone, troglitazone. Preferred pharmaceutically active ingredients from the thiazolidinone class are pioglitazone or rosiglitazone, more preferably pioglitazone. Pharmaceutically active ingredients from the biguanide class are for example phenformin, buformin or metformin, preferably metformin. Pharmaceutically active ingredients from the statin class are for example atorvastatin, cerivastatin, fluvastatin, lovastatin, mevastatin, pitavastatin, pravastatin, rosuvastatin or simvastatin. Preferred pharmaceutically active ingredients of the statin class are atorvastatin, pravastatin, rosuvastatin or simvastatin, more preferably rosuvastatin.
   In a further preferred embodiment, the pharmaceutical composition, powder or granulate according to the present invention comprises one or more pharmaceutically active ingredients according to item (11) and one or more pharmaceutically active ingredients according to item (12). In a further preferred embodiment, the pharmaceutical composition according to the present invention comprises one pharmaceutically active ingredient selected from the group according to item (11) and one pharmaceutically active ingredient selected from the group according to item (12).
(13) The pharmaceutical composition, powder or granulate according to any of items (2)-(11), wherein the constituents that form a solid dispersion or a solid solution are deposited on the pharmaceutically acceptable carrier.
   Preferably, the constituents form a solid dispersion.
(14) The pharmaceutical composition, powder or granulate according to any of the preceding items, wherein the pharmaceutical composition, powder or granulate is optionally mixed with a pharmaceutically acceptable excipient. Preferably, the excipients are fillers such as lactose, mannitol, cellulose derivatives, and sucrose; disintegrators such as crosscarmellose sodium, calcium carbonate, carboxymethylcellulose calcium, binders such as polyvinylpyrrolidone; lubricants such as magnesium stearate, stearic acid, and silicium dioxide; fragrants such as vanilla extract; colorants such as titanium dioxid; sweeteners such as saccharine; coating polymers such as hydroxypropylmethylcellulose; or vehicles such as water and organic solvents. Preferably, the pharmaceutical composition, powder or granulate do not contain a surfactant (surface active agent).
(15) Pharmaceutical dosage form comprising the pharmaceutical composition, powder or granulate according to any one of the preceding items, wherein preferably the pharmaceutical dosage form is a solid dosage form, preferably in the form of a pellet, a tablet, a capsule, a sachet, preferably the dosage form is a tablet.
(16) The pharmaceutical dosage form according to item (15), wherein the tablet is in a form of a monolayer, bilayer, multilayer tablet, and/or a coated tablet; and the capsule is a coated capsule.
(17) The pharmaceutical composition, powder, granulate or pharmaceutical dosage form according to any of items (2)-(17) wherein the pharmaceutically acceptable carrier is crystalline.
(18) A process for preparing a pharmaceutical composition comprising the steps of:
   a) providing at least one hyperbranched polymer, preferably as defined in item 3, and at least one active pharmaceutical ingredient, preferably at least one pharmaceutically active ingredient as defined in item (11) and/or (12), exhibiting a solubility in an aqueous medium at pH 6.8 and 37° C of less than 1 mg/ml, in a weight ratio of 99:1 w/w to 11:1 w/w, preferably in a weight ratio as defined in item 1, or wherein the weight ratio is 99:1 w/w to 4:1 w/w if the active pharmaceutical ingredient is selected from the group consisting of phosphodiesterase inhibitors, non-thiazide sulphonamides, sulfonylurea derivatives, and hydroxy-1,4-naphthoquinone derivatives, or wherein the weight ratio is 99:1 w/w to 4:1 w/w if a dendrimeric polyesteramide, preferably having tertiary amine or hydroxyl groups, is used as the hyperbranched polymer,
   b) forming a mixture of the at least one hyperbranched polymer, the at least one active pharmaceutical ingredient and an organic solvent, optionally mixing, and
   c) removing the solvent. Preferably, the at least one hyperbranched polymer and the at least one active pharmaceutical ingredient are completely dissolved in the organic solvent.
   Preferably, the organic solvent is removed in a first process forming a solid dispersion and/or a solid solution, preferably a solid dispersion. More preferably, the organic solvent is removed by using mixing, spray-drying, fluid-bed granulation or freeze drying, more preferably spray-drying and fluid-bed granulation.
(19) A process for preparing a powder or granulate according to the invention comprising the steps of:
   a) providing at least one hyperbranched polymer and at least one active pharmaceutical ingredient exhibiting a solubility in an aqueous medium at pH 6.8 and 37° C of less than 1 mg/ml, in a weight ratio of 99:1 w/w to 4:1 w/w,
   b) forming a mixture of the at least one hyperbranched polymer, the at least one active pharmaceutical ingredient and an organic solvent, and
   c) removing the solvent and preparing a powder or granulate, wherein step b) or step c) further comprises combining the mixture with a hydrophilic or hydrophobic carrier, preferably a hydrophilic carrier, more preferably a crystalline carrier. Preferably, the organic solvent is removed and effectively allows forming a solid solution and/or a solid dispersion, preferably a solid dispersion.
(20) The process according to item (18) or (19), wherein the organic solvent is selected from the group consisting of C₂-C₄ alkanols, such as ethanol, n-propanol, isopropanol, n-butanol, isobutanol; aliphatic or alicyclic ether, such as diethyl ether, diisopropyl ether, methyl tert-butyl ether, dioxane, tetrahydrofurane; ketone, such as acetone, methyl ethyl ketone; dimethylsulphoxide and dimethylformamide; or mixtures thereof.
(21) The process according to any of items (18)-(20), wherein in step b) a water-dispersible or water-soluble pharmaceutically acceptable carrier, preferably a carrier as defined in any of the above items, is added.
(22) The process according to any of items (18)-(21), wherein mixing can be carried out during step b) and or step c).
(23) The process according to any of items (18)-(22), wherein step c) is performed by using mixing, high shear mixing, spray drying, fluid bed granulation or freeze drying.
(24) The process according to any of items (18)-(23), further comprising a drying step. In a preferred embodiment, the drying can be carried out in a vacuum oven at a temperature of below 60°C, preferably below 50°C, most preferably at 40°C or below.
(25) The process according to any of items (20)-(26), wherein the powder or granulate is compressed into a tablet, or filled into a capsule.
(26) The process according to any of items (18)-(25), further comprising adding another pharmaceutically active ingredient. Preferably, the additional pharmaceutically active ingredient is selected from the thiazolidinone class, biguanide class, statin class, fibrate class, thiazide class, acarbose, miglitol, voglibose, orlistat, sibutramine, rimonabant, exenatide and pramlintide, it is preferably selected from biguanide class, thiazolidinone class and thiazide class, more preferably it is selected from thiazolidinone class.
(27) A process for preparing a solid dispersion and/or solid solution of a hyperbranched polymer and at least one pharmaceutically active ingredient comprising the steps of:
   a) forming a mixture of the at least one hyperbranched polymer, the at least one active pharmaceutical ingredient and an organic solvent, preferably the at least one hyperbranched polymer and the at least one active pharmaceutical ingredient are completely dissolved in the organic solvent,
   b) providing the solid dispersion and/or solid solution from the dispersion/solution of step a) by using high shear mixing, spray drying or fluid bed granulation, wherein the mixture in step a) or the solid dispersion and/or solid solution of step b) is optionally combined with a hydrophilic or hydrophobic carrier, preferably a hydrophilic carrier, more preferably a crystalline carrier.
(28) The process according to item (27), wherein the weight ratio of the at least one hyperbranched polymer and the at least one active pharmaceutical ingredient is defined as in the preceding items.
(29) Use of a crystalline carrier for the preparation of a pharmaceutical composition comprising hyperbranched polymer and a pharmaceutically active ingredient.
(30) Use according to item (29), wherein the hyperbranched polymer and the pharmaceutical ingredient form a solid dispersion.
(31) Use of dendrimeric polyesteramide hyperbranched polymers, preferably a dendrimeric polyester hyperbranched polymer having tertiary amine or hydroxyl end groups, for the preparation of a pharmaceutical composition comprising at least one active pharmaceutical ingredient, wherein the weight ratio of the hyperbranched polymer to the at least one active pharmaceutical ingredient is 99:1 w/w to 4:1 w/w.
(32) Use of a hydrophilic or hydrophobic carrier for the preparation of a pharmaceutical composition comprising a pharmaceutically active ingredient selected from the group consisting of phosphodiesterase inhibitors, non-thiazide sulphonamides, sulfonylurea derivatives, and hydroxy-1,4-naphthoquinone derivatives, preferably a non-thiazide sulphonamide, in mixture with at least one hyperbranched polymer.
(33) Use of a hydrophilic carrier for the preparation of a pharmaceutical composition comprising a pharmaceutically active ingredient selected from the group consisting of phosphodiesterase inhibitors, non-thiazide sulphonamides, sulfonylurea derivatives, and hydroxy-1,4-naphthoquinone derivatives, preferably a non-thiazide sulphonamide, in mixture with at least one hyperbranched polymer.
(34) Use of an active pharmaceutical ingredient selected from the group consisting of phosphodiesterase inhibitors, non-thiazide sulphonamides, sulfonylurea derivatives, and hydroxy-1,4-naphthoquinone derivatives for the preparation of a pharmaceutical composition comprising at least one hyperbranched polymer, wherein the at least one pharmaceutically active ingredient exhibits a solubility in an aqueous media at pH 6.8 and 37° C of less than 1 mg/ml, and wherein the weight ratio of the at least one hyperbranched polymer to the at least one active pharmaceutical ingredient is 99:1 w/w to 4:1 w/w.
(35) Use according to item (34), wherein the pharmaceutical ingredient is selected from the group consisting of pimobendan, tadalfil, indapamide, glimepiride, and atovaquone.

### Detailed description of the invention

The present invention is now described in more detail by preferred embodiments and examples, which are however presented for illustrative purpose only and shall not be understood as limiting the scope of the present invention in any way.

One of the most challenging problems in pharmaceutics is to deliver a therapeutically effective amount of a pharmaceutically active ingredient (active pharmaceutical ingredient, API) to the desired site of action. However, in order to exhibit the desired pharmacological activity, the API has to reach a sufficient concentration at the site of action. In order to allow an efficient API concentration to be reached at the desired site of action, the API should exhibit a solubility that allows an efficient concentration to be reached.

Accordingly, the pharmaceutical composition according to the present invention provides for the concentration of pharmaceutically active ingredient that is needed to be effective in the physiological environment. For instance upon oral administration of the pharmaceutical composition comprising the pharmaceutically active ingredient, the solubility of said API is sufficiently high to reach the required concentration at the target location, such as the gastrointestinal tract, or to achieve the necessary blood concentration upon absorption. Therefore, the pharmaceutical composition according to the present invention does not need to contain a larger amount of an API, which normally would be needed in order to compensate the poor solubility of the API in aqueous solution. This, in turn, leads to a pharmaceutical composition that does not exhibit an increased size, which could affect negatively patients' compliance. Furthermore, the pharmaceutical composition according to the present invention as well as the process for the preparation of such a pharmaceutical composition is much more cost effective: Based on the enhanced solubility of the API being present in the pharmaceutical composition, less amount of API is necessary in order to provide for the desired effective API concentration on the target site.

Moreover, by applying the process according to the present invention, loading restraints, toxicity (e.g. nephrotoxicity) of solubilizing agents, the requirement of maintaining a certain critical micellar concentration on surfactant based solubilization and limited choices in pH modifications can be avoided.

Within the context of the present invention it has been surprisingly observed that the solubility of poorly soluble active pharmaceutical ingredients can particularly be increased, provided that a specific weight ratio of the hyperbranched polymer and the at least one active pharmaceutical ingredient is used.

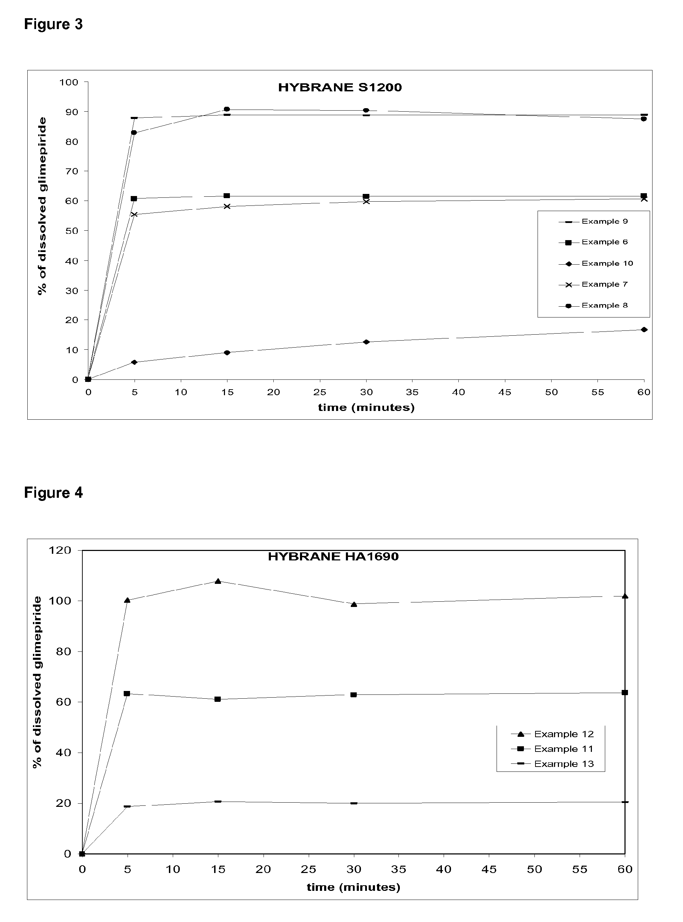

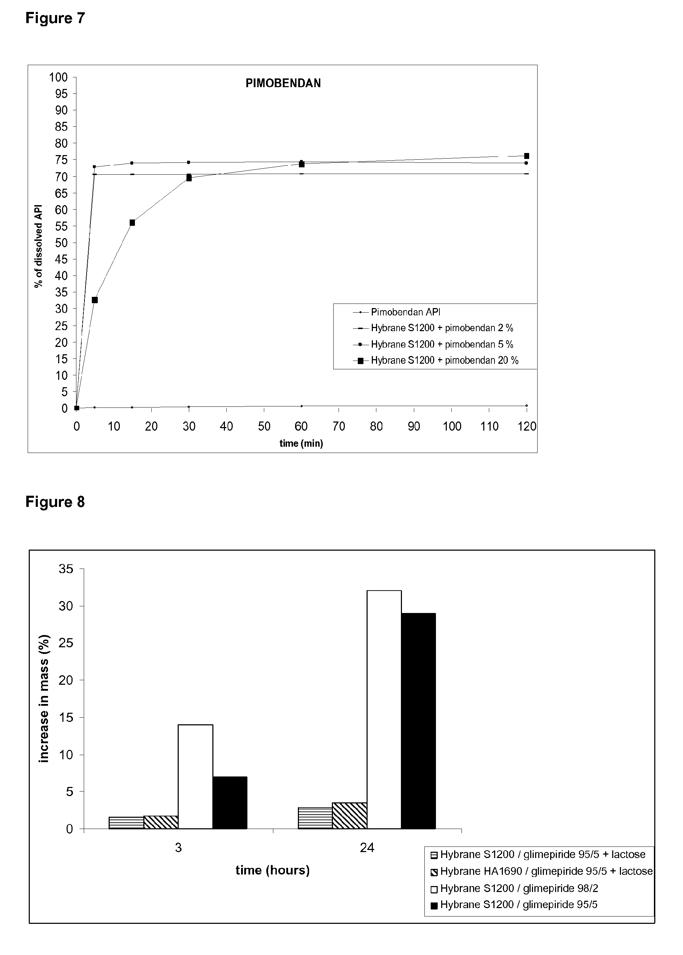

A particular high relative increase in solubility (compared to the API alone) can be obtained for the specific APIs as described herein. Among others, this contribution allows applying a more general weight ratio of hyperbranched polymer to API. As can be derived from Figures 3-7, the APIs according to the invention, which are selected from the group consisting of phosphodiesterase inhibitors, preferably phosphodiesterase III inhibitors or phosphodiesterase V inhibitors; non-thiazide sulphonamides; sulfonylurea derivatives; and hydroxy-1,4-naphthoquinone derivatives, particularly provide for a very high dissolution rate in the first 5 minutes of the dissolution test. Thus, mixtures of hyperbranched polymers with the APIs according to the invention provide for a rapid dissolution which leads to an advantageously fast increase of the drug plasma level in the subject to be treated with a pharmaceutical composition containing this mixture. A particularly preferred API is pimobendan which showed the highest increase of dissolution rate (compare Figure 7).

The solubility can be further increased if the hyperbranched polymer and the at least one active pharmaceutical ingredient form a solid dispersion. It has also been found that dendrimeric polyesteramide hyperbranched polymers, in particular dendrimeric polyesteramide hyperbranched polymers having tertiary amine or hydroxyl end groups, are particularly advantageous for increasing the solubility of an API (compare Figures 3 and 4). However, the use of a dendrimeric polyesteramide having hydroxyl end groups (e.g. Hybrane^{®}S1200) is preferred with respect to obtaining a pharmaceutical composition, powder, granulate or pharmaceutical dosage form having a very low hygroscopicity (see Figure 8 which shows the lowest water uptake if Hybrane^{®}S1200 is used as the hyperbranched polymer).

It has also been found that powders or granulates comprising a specific weight ratio of the hyperbranched polymer and the at least one active pharmaceutical ingredient and having improved properties (e.g. low hygroscopicity) can be prepared when using hydrophilic or hydrophobic carriers, preferably hydrophilic carriers (compare Figure 8). This alternative, or concurrent with API selection, technical contribution again allows applying a more general weight ratio of hyperbranched polymer to API. Moreover, it has further been observed that the stability and solubility of the API can be increased even more in case a crystalline carrier is used. This is in particular advantageous if the pharmaceutical composition should be in the form of a powder or granulate or a dosage form. It has been found that using crystalline carriers leads to a decreased hygroscopicity of the pharmaceutical composition, thereby avoiding the liquification of the powder or granulate. Furthermore, it has been unexpectedly found within the context of the present invention that the ratio of hyperbranched polymer/at least one active pharmaceutical ingredient should at least be 11:1 or 4:1 for specific APIs, in order to provide a significantly enhanced solubility. However, in case the amount of hyperbranched polymer is too high, the solubility decreases. Without wishing to be bound by any theory it is assumed that the solubility of the respective API increases because the previously unassociated, poorly soluble active pharmaceutical ingredient interacts with the hydroxyl, ester, amido and/or carboxyl groups of the hyperbranched polymers. In a weight ratio of hyperbranched polymer: API of more than 99:1, there might be a situation when H-bonds between the hyperbranched polymer itself predominates over the intermolecular interactions between the active ingredient and the polymer, leading to closing the polymer and not contributing any more to enhancing the solubility of the active ingredient. On the other hand, in case the ratio of hyperbranched polymer/pharmaceutically active ingredient is 11:1 or more, the interactions between the molecules of the pharmaceutically active ingredients can lead to a crystallization of the pharmaceutically active ingredient which reduces the solubility.

One of the most important points that have to be fulfilled when designing pharmaceutical compositions is that the constituents of said composition are biocompatible and suitable to be used in living organisms.

As can be seen for example for many cationic systems, including liposomes and micelles with surface groups bearing cationic charge, these cationic systems can readily damage cell membranes, leading to cell lysis. Hence, the issue of low-toxicity is relevant for hyperbranched polymers, as for any other prospective pharmaceutical excipient, before considering their use. Due to the biocompatible properties of the hyperbranched polymers, they are suitable for use in mammals. In particular hyperbranched polyesteramides have both amides as well as ester linkages which attributes to amphiphilicity and biodegradability. Hyperbranched polyesteramides possess the excellent mechanical properties of polyamides and the biodegradability of polyester. Owing to the polar nature of amide groups and their ability to form hydrogen bonds, these polymers exhibit good thermal and mechanical properties even at low molecular weight. On the other hand, hydrolytically degradable ester bond provides biodegradability to the polymer. Branching of polyesteramides have been reported to substantially enhance hydrolysis both in alkaline and in phosphate buffered saline solution, exceptionally reducing the burden to the living organism.

In the following, the pharmaceutical compositions, powders or granulates are described in more detail. The pharmaceutical compositions, powders or granulates comprise at least one hyperbranched polymer, preferably the specific hyperbranched polymers as described herein.

Within the meaning of the present invention, the term "hyperbranched polymer" generally denotes very generally polymers known as dendrimers, arborol, cascade, cauliflower or star polymers, or other high molecular weight polymers, which all have specific branched structure containing a center atom or a molecule, which can be monomeric or polymeric, from where three or more chains emanate. Hyperbranched polymers are thus distinguishable from linear polymers, copolymers or graft polymers, crosslinked linear polymers or comb polymers.

Another remarkable characteristic of hyperbranched polymers within the meaning of the invention is that they possess a large number of terminal functional groups. When having a large number of terminal groups, intermolecular interactions between the hyperbranched polymers depend largely on the types of the terminal functional groups, resulting in large variations in the glass transition temperature, the solubility, the thin film forming property, or the like. In particular, due to the large number of terminal groups, such a hyperbranched polymer has characteristics that no general linear polymer has. The hydrodynamic characteristics of hyperbranched polymers significantly differ from those of linear polymers with similar chemical structure of repeating units and the same molecular weight. One of the most important characteristics of hyperbranched or dendritic polymers is their inability to crystallize or entangle the polymer chain regardless of their molecular weights. The miscibility of these polymers cannot be accurately predicted with current understanding on the rules of linear polymer, since the branched structure affects interpolymer chain interaction. In addition, the hyperbranched polymer can have a large number of functional groups also along the branched chains. According to the invention, it is preferred to use hyperbranched polymer comprising hydroxyl groups, ester groups, amido groups and/or carboxyl groups as terminal functional groups (end groups). Preferable, the hyperbranched polymer comprises either ester groups or amido groups or both, further preferred the hyperbranched polymer is a dendrimeric polyesteramide, further preferred dendrimeric polyesteramides having tertiary amine end groups.

In general, hyperbranched polymers can be synthesized according to any suitable method that is known to a person skilled in the art. Preferably, the hyperbranched polymers are synthesized by coupling the same or different monomers to the center atom or a molecule in one or more runs, wherein at least some monomers have three or more functional groups, making subsequent coupling reactions possible at the unreacted terminal functional groups, which leads to the formation of branched extensions. A monomer within the meaning of the present invention is a small molecule that can become chemically bonded to other monomers in order to form polymers. Monomers can be either synthetic, such as hydrocarbons, or natural, such as amino acids. The aforementioned method can be used to generate different degrees of random or ordered hyperbranched polymers. Further, the terminal functional groups of the hyperbranched polymers can be tailored by subsequent substitution or addition reactions for reasons such as to increase the number of functional groups or to substitute them, and covalently link the polymer with another molecule, i.e. for delivery of nutriments, vitamins, or active pharmaceutical ingredients. Furthermore, the use polyethylenglycols as polymer chains allows for protecting the hyperbranched polymer from being detected by the mononuclear phagocyte system when administered to humans or animals. It is also possible to introduce recognizable groups complementary to a receptor or to a tissue, or to introduce moieties facilitating the transport of the hyperbranched polymer through the cell membrane.

Preferred hyperbranched polymers according to the present invention are hyperbranched polyimines, hyperbranched polyurethanes, hyperbranched polyamides, hyperbranched polyesteramines, hyperbranched polyesteramides, particulary preferred are hyperbranched polyesteramines and hyperbranched polyesteramides, even further preferred are hyperbranched polyesteramides. It is understood that said hyperbranched polymers can have different monomers introduced in their structure and the terminal functional groups can optionally be modified.

The pharmaceutical compositions, powders or granulates further comprise at least one active pharmaceutical ingredient having a low solubility.

Numerous active pharmaceutical ingredients have a solubility in an aqueous media at pH 6.8 and 37° C of less than 1 mg/ml. Solubility as meant herein is measured using USP apparatus 2 (U.S. Pharmacopoeia 32-NF27) with agitation at 75 RPM in the dissolution medium of a phosphate buffer maintained at pH 6.8 and 37° C. Such low solubility makes preparing a useful pharmaceutical composition of acceptable size and efficacy especially difficult.

The pharmaceutical composition according to the present invention contains at least one pharmaceutically active ingredient exhibiting solubility in aqueous media at pH 6.8 and 37°C of less than 1 mg/ml. In a preferred embodiment, this is irrespective of the pharmacological effect it might have or ailment it is used to prevent or treat. When defining the active pharmaceutical ingredient to be used according to the invention, the solubility of this active pharmaceutical ingredient is measured by using only the active pharmaceutical ingredient, i.e. the active pharmaceutical ingredient is not mixed with any additives like e.g. hyperbranched polymers. In a preferred embodiment according to the present invention, the solubility of the active pharmaceutical ingredient in an aqueous media at pH 6.8 and 37° C is in a range of equal to or less than 1 mg/ml and equal to or more than 0.2 mg/ml, further preferred in a range of equal to or less than 1 mg/ml and equal to or more than 0.3 mg/ml, even further preferred in a range of equal to or less than 1 mg/ml and equal to or more than 0.4 mg/ml.

Preferred active pharmaceutical ingredients are selected from the group consisting of phosphodiesterase inhibitors, preferably phosphodiesterase III inhibitors or phosphodiesterase V inhibitors; non-thiazide sulphonamides; sulfonylurea derivatives; and hydroxy-1,4-naphthoquinone derivatives. A preferred phosphodiesterase III inhibitor is pimobendan, a preferred phosphodiesterase V inhibitor is tadalfil; a preferred non-thiazide sulphonamide is indapamide; preferred sulfonylurea derivatives that are insulin secretion enhancers are glimepiride, gliclazide, glipizide and glibenclamide; and a preferred hydroxy-1,4-naphthoquinone derivative is atovaquone. It has been unexpectedly found that the relative solubility (relative to the solubility of the API alone) can be more enhanced if the aforementioned APIs are used, particularly in combination with the preferred hyperbranched polymers and the preferred weight ratio of hyperbranched polymer/API as described herein.

It has been unexpectedly found that the solubility of the aforementioned APIs, preferably that of phosphodiesterase III inhibitors, e.g. pimobendan, can be particularly strongly increased, even if the weight ratio of hyperbranched polymer/API is only 4:1 or higher, for example 50:1 (compare Figure 7). Pimobendan and levosimendan are selective phosphodiesterase III inhibitors used in the management of heart failure.

Tadalafil is a phosphodiesterase E5 inhibitor and can be used for treating erectile dysfunction or pulmonary arterial hypertension. Atovaquone is known of its anti-yeast and antiparasitic activity.

It has also been unexpectedly found that the solubility of non-thiazide sulphonamides (diuretic compounds), preferably indapamide, can be particularly increased if a hydrophilic or hydrophobic carrier, preferably a hydrophilic carrier, is used for the preparation of a pharmaceutical composition comprising the non-thiazide sulphonamide in mixture with at least one hyperbranched polymer.

The solubility in aqueous media of pH around 7 of the respective compounds is as follows: glimepiride 0.001 mg/ml; gliclazide 0.05 mg/ml; glipizide 0.037 mg/ml; glibenclamide 0.06 mg/ml; pimobendan 0.21 mg/ml; tadalafil 0.11 mg/ml; atovaquone 0.003 mg/ml; indapamide 0.7 mg/ml. In a preferred embodiment according to the present invention, the increase in solubility of the active ingredient in aqueous media under the above indicated conditions is 10, 50, 55, 100 and 140-fold, respectively, compared to the solubility of the pure API. In particular, the highest increase in solubility can be achieved if the active pharmaceutical ingredient is selected from the group consisting of phosphodiesterase inhibitors, non-thiazide sulphonamides, sulfonylurea derivatives, and hydroxy-1,4-naphthoquinone derivatives, an even further increase can be achieved when using glimepiride, pimobendan, tadalafil, atovaquone or indapamide as API. Although not wishing to be bound to any theory, it is assumed that this is due to intermolecular H-bonds that form between molecular groups of API and interior carboxyl groups of the hyperbranched polymer.
The pharmaceutical compositions, powders or granulates according to the invention comprise the hyperbranched polymer/API in specific weight ratios.

Preferably, the weight ratio of hyperbranched polymer/active pharmaceutical according to the invention is 99:1 to 11:1. In a preferred embodiment, the weight ratio is 99:1 to 14:1, further preferred the weight ratio is 99:1 to 17:1, even further preferred, the weight ratio is 99:1 to 18:1. In another preferred embodiment, the weight ratio is 80:1 to 11:1, further preferred, the weight ratio is 55:1 to 11:1, even further preferred, the weight ratio is 24:1 to 11:1. In a further embodiment, the weight ratio is 80:1 to 14:1, further preferred, the weight ratio is 55:1 to 17:1, even further preferred, the weight ratio is 24:1 to 18:1 Preferably, for example when the active pharmaceutical ingredient is glimepiride or indapamide, the weight ratio of polybranched polymer and said active pharmaceutical ingredient is 98:2 w/w to 95:5 w/w, further preferred 98:2 w/w to 95:2 w/w. As can be seen from the experimental data contained herein, the use of a weight ratio of 99:1 to 11:1 provides for a higher solubility compared to the use of a weight ratio of 4:1 (compare Figures 3-5). The figures also show that the highest increase in solubility can be achieved when using a weight ratio of 19:1.

In general, the weight ratio of hyperbranched polymer/API should be 11:1 or above. However, as indicated above, it has been found that the solubility of the specific active pharmaceutical ingredients selected from the group consisting of phosphodiesterase inhibitors, non-thiazide sulphonamides, sulfonylurea derivatives, and hydroxy-1,4-naphthoquinone derivatives can also be highly increased when using a weight ratio of hyperbranched polymer/API of as low as 4:1 (or above). Furthermore, the weight ratio can be as low as 4:1 (or above) if the specific dendrimeric polyesteramide hyperbranched polymers as described herein are used. The preferred weight ratios of 11:1 and above as defined herein, however, represent the preferred embodiments of the invention independent of the constituents which are used.

The pharmaceutical compositions, powders and granulates comprising at least one hyperbranched polymer and at least one pharmaceutically active ingredient exhibiting solubility in an aqueous media at pH 6.8 and 37° C of less than 1 mg/ml can be prepared by providing both constituents in an organic solvent in the specific weight ratios as defined herein and removing the solvent. It has been found that the constituents get intimately mixed and thus probably develop the highest possible number of intermolecular interactions between the polymer and the active ingredient if the solid dispersion is provided from the dispersion/solution of the at least one hyperbranched polymer and the at least one API in the organic solvent by using known techniques such as for example high shear mixing, spray drying or fluid bed granulation. It is particularly preferred that the API is completely dissolved in the organic solvent. As described herein, it is preferred to use a hydrophilic or hydrophobic carrier, specifically a hydrophilic carrier, in particular if a powder or granulate comprising the API is prepared. Depending on whether a solution or dispersion, e.g. a dispersion containing solid API and/or solid carrier particles, is used for preparing a mixture or solid dispersion according to the invention, a person skilled in the art considering the teaching provided herein can choose a suitable method for preparation.

Within the meaning of the present invention, the term "solid dispersion" refers to a homogenous mixture of active pharmaceutical ingredient (particles) in the hyperbranched polymer. Active pharmaceutical ingredient is dissolved or dispersed in the polymer. In general, a solid solution is a solid-state solution of one or more solutes in a solvent (in this case the "solvent" is represented by the hyperbranched polymer). Such a mixture is considered a solution rather than a compound when the structure of the solvent remains unchanged by addition of the solutes, and when the mixture remains in a single homogeneous phase. Thus, the at least one hyperbranched polymer and the at least one active pharmaceutical ingredients, are in contact with each other, preferably over a large surface area. The solid dispersion of components can be obtained by using the processes as described herein. Preferably, the solid dispersion of the active pharmaceutical ingredient and hyperbranched polymer is obtained by using the solvent method, wherein both the active pharmaceutical ingredient and the hyperbranched polymer are completely dissolved in an organic solvent, wherein upon removal of the organic solvent the solid dispersion is obtained. Techniques allowing fast removal of the organic solvent appear to assist in the formation of association and/or complex contacts between the polymer and the API, optionally further enhanced by the presence of hydrophilic or hydrophilic carrier, particularly by the presence of hydrophilic carrier. Preferably, removal of the organic solvent is accomplished over multiple days, preferably over 24 hours, more preferably within 10 hours and most preferably within 1 hour. Without being bound to any theory, it is believed that API-API association, API secondary particle formation, and API crystallization can be inhibited by such fast solvent removal, all such phenomena otherwise counter-acting the desirable association and/or complexation as disclosed herein. The removal of the solvent is efficiently carried out by using known techniques like mixing, high shear mixing, spray drying, fluid bed granulation or freeze drying. Preferably, high shear mixing, spray drying or fluid bed granulation are used. When providing the solid dispersion on solid carrier particles, the solution or dispersion of hyperbranched polymer/API is preferably sprayed onto the carrier particles by using known techniques.

Mostly, hyperbranched polymers are water soluble through terminal functional groups and are extremely hygroscopic because of their amorphous form. When a hygroscopic hyperbranched polymer is exposed to the atmosphere, water from vapour migrates into/onto the polymer, and some of the water molecules bind to the polymer chains by intermolecular forces. Hygroscopicity of a hyperbranched polymer can be measured by methods known to a person skilled in the art. Mainly, such methods comprise determining the mass of a small amounts of a sample (i.e. 5-10 mg) as a function of time, while varying the controlled relative humidity and/or temperature in the environment. Upon exposure to average room relative humidity the hygroscopic nature of the hyperbranched polymer causes the pharmaceutical composition comprising the hyperbranched polymer and the active pharmaceutical ingredient to attract water and, e.g. when having a pharmaceutical composition in powder form, the particles of the powder change shape and eventually cause the composition to liquefy. However, it is desired that the properties of a pharmaceutical composition, which is e.g. in the form of a powder, do not change upon storage.

The pharmaceutical compositions according to the invention preferably comprise a carrier. The powders or granulates according to the invention also comprise a carrier.

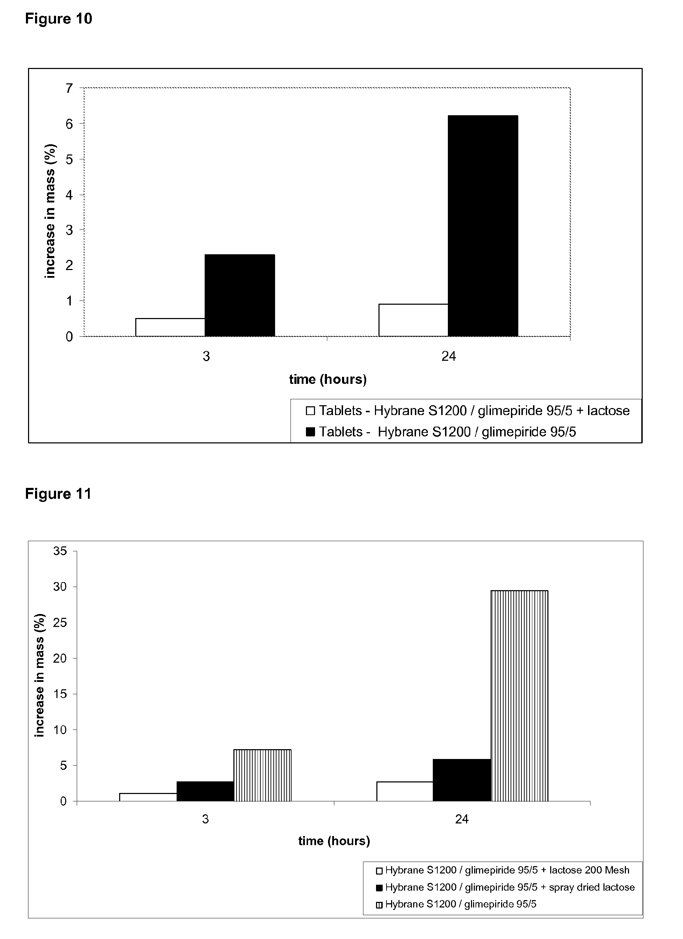

It has now been unexpectedly found that adding a water-dispersible or water-soluble pharmaceutically acceptable carrier, preferably a hydrophilic or hydrophobic carrier, more preferably a hydrophilic carrier, particularly preferable a crystalline carrier, to the pharmaceutical composition significantly reduces the water take-up of the pharmaceutical composition from the atmosphere and retains its original properties, e.g. the powdery form (see e.g. Fig. 11). Particularly preferred is a crystalline hydrophilic carrier. The properties of the inventive pharmaceutical compositions remain steady in terms of adequate flowability, chemical stability and compressibility, in particular when using a crystalline carrier. Flowability, chemical stability or compressibility can be determined by using known methods in the art, for example the methods explained in the European Pharmacopoeia, 6th Edition. With respect to chemical stability it has been surprisingly observed that no significant degradation of otherwise water sensitive API, such as for example glimepiride, occurs when the water-dispersible or water-soluble carrier, preferably a hydrophilic, preferably a crystalline, carrier, is added to the pharmaceutical composition of the present invention even if thus obtained pharmaceutical composition is exposed to elevated relative humidity of 75 %. The same applies to a dosage form disclosed according to the present invention. The presence of the carrier also provides that the pharmaceutical composition is suitable for molding processes. The use of carriers, in particular crystalline carriers, in powders, granulates, pellets, pills, tablets, capsules thus increases the shelf life of the dosage form.

Within the meaning of the present invention the "water-dispersible or water-soluble pharmaceutically acceptable carrier" is construed as any pharmaceutically acceptable excipient in a solid or semi-solid form, which can be dispersed or dissolved in water at 37 °C

Said carrier can be for example a modified or unmodified carbohydrate, which can be monomeric, oligomeric or polymeric; a wax; a gum; an organic or inorganic acid or base, or salt thereof, a surfactant, a synthetic polymer, a modified or unmodified silica, a mineral pharmaceutical compound such as calcium phosphate, calcium sulfate, calcium chloride, magnesium carbonate, magnesium oxide, sodium chloride, magnesium aluminum silicate, polacrilin potassium, talc, aluminum hydroxide, aluminum oxide, or a combination thereof.

Said carrier can also be for example carboxymethylcellulose calcium, carboxymethylcellulose sodium, cellulose acetate, cellulose acetate phthalate, ceresin, cetyl alcohol, chitosan, cellulose, ethylcellulose, gelatin, glucose, glyceryl behenate, glyceryl palmitostearate, methylcellulose, hydroxyethyl cellulose, hydroxyethylmethyl cellulose, microcrystalline cellulose, silicified microcrystalline cellulose, hydroxypropyl cellulose, hypromellose, hypromellose phthalate, croscarmellose sodium, hydroxypropyl cellulose, isomalt, maltitol, mannitol, kaolin, lactitol, maltodextrin, polydextrose, dextrates, dextrin, dextrose, methylcellulose, polydextrosepoly(dl-lactic acid), polyethylene oxidepolyvinyl, acetate phthalate, polyvinyl alcohol, shellac, sucrose, fructose, maltose, trehalose, sucrose octaacetate, sugar, lactose, cyclodextrin, corn starch, pregelatinized starch, hydroxypropyl starch, titanium dioxide, wax (carnauba), wax (microcrystalline), xylitol, zein, copovidone, simethicone, crospovidone, erythritol, glyceryl palmitostearate, sorbitolsulfobutylether β-cyclodextrin, vitamin e polyethylene glycol succinate, acacia, agar, guar gum, carbomer, gelatin, pectin, ceratonia, chitosan, copovidone, polyethylene oxide, polymethacrylates, povidone, glyceryl behenate, inulin, cetylpyridinium chloride, glyceryl monostearate, hydroxypropyl betadex, lecithin, macrogol 15 hydroxystearate, phospholipids, poloxamer, polyoxyethylene alkyl ethers, polyoxyethylene castor oil derivatives, polyoxyethylene sorbitan fatty acid esters, polyoxyethylene stearates, polyoxylglycerides, pyrrolidone, sorbitan esters (sorbitan fatty acid esters), sulfobutylether β-cyclodextrin, tricaprylin, triolein, vitamin e polyethylene glycol succinate, wax, benzalkonium chloride, isoleucine, alanine, leucine, asparagine, lysine, aspartate, methionine, cysteine, phenylalanine, glutamate, threonine, glutamine, tryptophan, glycine, valine, proline, serine, tyrosine, arginine, histidine, stearic acid, citric acid, malic acid, pentetic acid, adipic acid, alginic acid, boric acid, calcium lactate, calcium phosphate, citric acid monohydrate, maleic acid, monosodium glutamate, potassium citrate, sodium acetate, sodium alginate, sodium borate, sodium carbonate, sodium citrate dihydrate, sodium hydroxide, sodium lactate, sodium hydrogenphosphate, sodium dihydrogenphosphate, sodium starch glycolate, calcium carbonate, calcium lactate, calcium phosphate, calcium sulfate, calcium chloride, magnesium carbonate, magnesium oxide, sodium chloride, magnesium aluminum silicate, polacrilin potassium, talc, aluminum hydroxide, aluminum oxide, aluminum phosphate adjuvant, attapulgite, bentonite, calcium silicate, colloidal silicon dioxide, hectorite, kaolin, magnesium aluminum silicate, magnesium carbonate, polycarbophil, saponite, magnesium aluminum silicate, or a mixture thereof. Preferably, the carrier is a hydrophilic carrier, which is preferably selected from the group consisting of sugars and carbohydrates, preferably the carrier is selected from the group consisting of modified or unmodified monosaccharides, straight or branched oligosaccharides, and straight or branched polysaccharides, or mixtures thereof. Further preferred, the carrier is selected from the group consisting of lactose, sucrose, maltose, mannitol, sorbitol, xylitol, erythritol, lactitol, maltitol, starch, or cellulose, preferably lactose, maltose, mannitol, sorbitol, starch and cellulose, more preferably lactose and cellulose, yet more preferably is lactose.

Another advantage of adding a pharmaceutically acceptable carrier to the pharmaceutical composition is that the carrier contributes to the formation a powder or granulate. The amount of carrier included in the pharmaceutical composition should preferably be from 10 % to 90 %, preferably from 30 % to 60 %, more preferably from 50 % to 80 %, yet more preferably from 70 % to 80 % based on the total weight of the pharmaceutical composition. The carrier prevents the composition from increasing in weight due to water absorption of water originating from the environment, e.g. air. Moreover, the carrier prevents the particles of the composition from growing in size and enables it to be used essentially unchanged over time. The beneficial effect of limited size distribution is improved flowability, particularly when the invention is practiced on a large scale.

For pharmaceutical compositions in powder or granulate form, a hydrophilic carrier as defined above is used. The carrier can be in particulate form and/or crystalline form.

Particularly advantageous effects can be provided when the carrier is used in crystalline form. The protective effect towards the composition in this case is most pronounced, probably because the carrier in crystalline form is less subjected to hygroscopicity. It has been unexpectedly found that the hygroscopicity is reduced even if the solid dispersion according to the invention is deposited on the carrier surface. Thus, it is preferred to deposit the solid dispersion onto the carrier surface. This is achieved by e.g. using the solvent method as described herein, wherein the hyperbranched polymer and the pharmaceutically active ingredient are firstly dissolved in an organic solvent, whereupon the carrier, preferably a crystalline carrier, is added to the solution and then the solvent is removed (solvent method). Alternatively, the carrier is added to the hyperbranched polymer and the pharmaceutically active ingredient only after the solvent has been already removed, or at least partly removed. This can be achieved simply by mixing obtained solid dispersion comprising the hyperbranched polymer and the pharmaceutically active ingredient with the carrier. Additional option is to remove solvent while the carrier is being combined with the two constituents. An example where this takes place is when the solution/dispersion of hyperbranched polymer and API is sprayed onto the carrier particles, leading to evaporation and thus removal of the solvent while the carrier is being coated by the arising solid dispersion. The skilled person would understand that the order or combination of steps where the carrier is added can be chosen as desired, i.e. the carrier can be added simultaneously or sequentially to providing the at least one hyperbranched polymer and the at least one pharmaceutically active ingredient to organic solvent or to removing the solvent. The carrier, preferably in crystalline form, can be even first added into the solution/dispersion and then again after solvent removal. Preferably, the solution/dispersion of hyperbranched polymer and API is sprayed onto the carrier particles as described herein.

In order to determine whether or not a pharmaceutical composition comprises a crystalline carrier, X-ray powder diffraction analysis can be carried out. In case the X-ray data show the peaks of the crystalline carrier material, the pharmaceutical composition contains a crystalline carrier. In case the X-ray data do not show any peaks, the pharmaceutical composition is amorphous and does not represent a pharmaceutical composition according to the preferred embodiment of the invention. Characteristic peaks of a crystalline carrier can be easily obtained from publicly available data bases by a person skilled in the art. It is also possible to first carry out an X-ray powder diffraction analysis of the crystalline carrier itself in order to identify the characteristic peaks of the carrier material. In case an unknown pharmaceutical composition is analyzed, no crystalline carrier is present in case no peaks can be determined. In case peaks are determined, e.g. the pharmaceutical composition can firstly be analyzed to find out which specific carrier material has been used. The characteristic peaks of this carrier material can then be obtained from literature or routine experiments.

According to the present invention, additional excipients can be used. Such excipients can be added together with the carrier, in particular prior to the solvent removal (according to the solvent method as described herein) or after the mixture, preferably solid dispersion, of hyperbranched polymer and API has been prepared in a solid form. Pharmaceutical excipients which also represent carriers according to the above definition will be referred to as carriers in case they are added to the mixture prior to solvent removal. In case such excipients are used after the preparation of the solid dispersion, e.g. if the excipients are added to the coating of a tablet, such excipients are not regarded as carriers. On the other hand, some of the carriers as indicated above might also be suitable as pharmaceutical excipients which are used after the solvent removal.

Suitable excipients are any conventionally used pharmaceutically acceptable excipients. For example, the excipients can be fillers (lactose, mannitol, cellulose derivatives, sucrose, etc.), disintegrators (crosscarmellose sodium, calcium carbonate, carboxymethylcellulose calcium etc.), binders (polyvinylpyrrolidone, etc.), lubricants (magnesium stearate, stearic acid, silicium dioxide, etc.), fragrants, colorants (titanium dioxide, etc.), sweeteners (saccharine, etc.), coating polymers (hydroxypropylmethylcellulose, etc.), or vehicles (water, organic solvents, etc.).

In a further embodiment, the pharmaceutical compositions, powders or granulates further comprise another active pharmaceutical ingredient. Such an additional active ingredient may be chosen to complement the pharmacological activity of the first active pharmaceutical ingredient, such as those mentioned above, in terms of providing an additive effect, a synergistic effect, a complementary effect, reduction of side effects, or the like.

Preferably the additional active pharmaceutical ingredient is selected from the thiazolidinone class, biguanide class, statin class, fibrate class, thiazide class, acarbose, miglitol, voglibose, orlistat, sibutramine, rimonabant, exenatide and pramlintide, it is preferably selected from biguanide class, thiazolidinone class and thiazide class, more preferably it is selected from thiazolidinone class. Active pharmaceutical ingredients from the thiazolidinone class are for example pioglitazone, rivoglitazone, rosiglitazone, troglitazone. Preferred active pharmaceutical ingredients from the thiazolidinone class are pioglitazone or rosiglitazone, more preferably pioglitazone. Active pharmaceutical ingredients from the biguanide class are for example phenformin, buformin or metformin. A preferred biguanide class active pharmaceutical ingredient is metformin. Active pharmaceutical ingredients from the statin class are for example atorvastatin, cerivastatin, fluvastatin, lovastatin, mevastatin, pitavastatin, pravastatin, rosuvastatin or simvastatin. Preferably statin class active pharmaceutical ingredients are atorvastatin, pravastatin, rosuvastatin or simvastatin, more preferably rosuvastatin.

Active pharmaceutical ingredients from the fibrate class are for example bezafibrate, ciprofibrate, clofibrate, gemfibrozil or fenofibrate. Preferred fibrate class active pharmaceutical ingredients are gemfibrozil or fenofibrate, more preferably fenofibrate. Active pharmaceutical ingredients from the tiazide class are for example chlorothiazide, or hydrochlorothiazide, preferably hydrochlorothiazide.

In a preferred embodiment of the present invention, the pharmaceutical composition, powder or granulate comprises at least one active pharmaceutical ingredient selected from the group consisting of glimepiride, gliclazide, glipizide, glibenclamide, pimobendan, tadalafil, atovaquone and indapamide and at least one hyperbranched polymer, wherein the at least one active pharmaceutical ingredient and hyperbranched polymer are mixed, preferably intimately mixed, at a weight ratio as indicated above, e.g. in a weight ratio of 99:1 w/w to 11:1 w/w, preferably of 98:2 w/w to 95:2 w/w. In another specific embodiment the at least one active pharmaceutical ingredient is glimepiride, pimobendan, tadalafil, atovaquone or indapamide, preferably is glimepiride or pimobendan, more preferably is glimepiride. In yet more preferable embodiment said pharmaceutical composition further comprises a water-dispersible or water-soluble pharmaceutically acceptable carrier, preferably lactose.

One embodiment of the present invention relates to a powder or granulate comprising
a) at least one hyperbranched polymer,
b) at least one pharmaceutically active ingredient exhibiting solubility in an aqueous media at pH 6.8 and 37° C of less than 1 mg/ml,
   wherein the weight ratio of the at least one hyperbranched polymer to the at least one active pharmaceutical ingredient is 99:1 w/w to 4:1 w/w, and
c) a hydrophilic or hydrophobic carrier, preferably a hydrophilic carrier, more preferably the carrier is crystalline.

Preferably, the hyperbranched polymer and the at least one pharmaceutical ingredient form a solid dispersion. The powder or granulate can be prepared by using mixing, high shear mixing, and/or spray drying, fluid bed granulation or freeze drying. Preferably it is prepared by using high shear mixing, and/or spray drying or fluid bed granulation. The preferred hyperbranched polymers, preferred APIs, preferred carriers, and preferred weight ratios of hyperbranched polymers/API etc. are described above.

The aforementioned pharmaceutical compositions, powders and granulates are suitable for preparing a medicament.

Thus, the present invention also relates to a pharmaceutical dosage form comprising the pharmaceutical composition, powder or granulates according to any one of the preceding items, wherein preferably the pharmaceutical dosage form is a solid dosage form, preferably in the form of a pellet, a tablet, a capsule, a sachet, preferably the dosage form is a tablet. The tablet can have one layer or can be a double or a multilayer tablet, which is preferably a film coated, or mantle coated tablet.

Preferably, the pharmaceutical composition, powder or granulate and pharmaceutical dosage form does not contain a surfactant.

The present invention also refers to processes for preparing the inventive pharmaceutical compositions, powders, granulates and pharmaceutical dosage forms.

The processes according to the invention can be carried out using the specific hyperbranched polymers, APIs, carriers, preferred weight ratios of hyperbranched polymers/API etc. as described above.

Basically, the process according to the invention in a first step comprises the addition of the at least one hyperbranched polymer and the at least one active pharmaceutical ingredient to an organic solvent. Preferably, the obtained solution or dispersion is mixed. Then, optionally an at least one carrier is added. In order to provide a solid pharmaceutical composition, the solvent is removed. The carrier can be alternatively combined with both constituents only after the solvent is removed or while it is being removed.

The removal of the solvent can be carried out by using standard techniques. Preferably techniques are applied that sufficiently remove the solvent already at the relatively low temperatures. The step of removing the solvent is preferably accomplished at the temperatures of up to 60 °C, more preferably of up to 50 °C. This further enhances the stability of the composition. During the solvent removal, the pharmaceutically active ingredient(s) can solidify and can, at least partially, but preferably completely also form a solid solution with the hyperbranched polymer. The process of solvent removal allows the constituents to become amorphous, which also assists in increasing solubility, especially when performed in relative short periods of time by suitable techniques such as spray-drying, fluid-bed granulation or freeze drying, preferably spray-drying or fluid-bed granulation. Preferably, the process according the invention further comprises a drying step to remove residual water and organic solvents. The temperature used in the drying process is preferably kept below 60°C, preferably below 50°C. In a preferred embodiment, the drying can be carried out in a vacuum oven at a temperature of below 60°C, preferably below 50°C, most preferably at 40°C or below.

In all processes according to the invention, preferably the at least one hyperbranched polymer and the at least one active pharmaceutical ingredient is completely dissolved in the organic solvent. If a carrier is used, the carrier can be either combined with the hyperbranched polymer and the API before the solvent is removed or after solvent removal. Another possibility is to spray the solution or dispersion of the at least one hyperbranched polymer and the at least one API onto the carrier as described below. In the latter case the combining of the carrier and the API/hyperbranched polymer and the solvent removal takes place in the same step. Also possible is to remove the solvent in step c) by using high shear mixing, spray drying, fluid bed granulation or freeze drying as described herein. In this case a solid dispersion of the API/hyperbranched polymer on the carrier is obtained. If no carrier is used, the solvent in step c) can also be removed by using mixing, high shear mixing, spray drying, fluid bed granulation, freeze drying or the like as described herein to provide a solid dispersion of the constituents.

The processes according to the invention can be used for preparing solid pharmaceutical dosage forms as well as gels or solutions. The solvent is removed in order to provide the mixture, preferably solid dispersion as a solid, preferably as a powder or granulate. When preparing gels or solutions, the solid obtained after removal of the solvent can e.g. be redissolved in a pharmaceutically acceptable solvent or buffer solution.

The solvents suitable for preparing the pharmaceutical compositions can be any solvent that at least partially dissolves both constituents (API and hyperbranched polymer). Preferably, the organic solvent is selected from the group consisting of C₂-C₄ alkanols, such as ethanol, n-propanol, isopropanol, n-butanol, isobutanol; aliphatic or alicyclic ether, such as diethyl ether, diisopropyl ether, methyl tert-butyl ether, dioxane, tetrahydrofurane; ketone, such as acetone, methyl ethyl ketone; dimethylsulphoxide and dimethylformamide; or mixtures thereof. The constituents in the solvent can be optionally mixed.

In a specific embodiment of the invention, at least 50 % w/w of both constituents get dissolved in the solvent, preferably more than 80 % w/w, more preferably over 90 % w/w, yet more preferably get completely dissolved in the solvent.

One process for preparing a pharmaceutical composition according to the invention comprises the steps of:
a) providing at least one hyperbranched polymer and at least one active pharmaceutical ingredient exhibiting a solubility in an aqueous medium at pH 6.8 and 37° C of less than 1 mg/ml, in a weight ratio as defined above,
b) adding the at least one hyperbranched polymer and the at least one active pharmaceutical ingredient to an organic solvent, and
c) removing the solvent.

The process for preparing a powder or granulate according to the invention comprises the steps of:
a) providing at least one hyperbranched polymer, preferably a specific hyperbranched polymer as defined above, and at least one active pharmaceutical ingredient, preferably at least one specific pharmaceutically active ingredient as defined above, exhibiting a solubility in an aqueous medium at pH 6.8 and 37° C of less than 1 mg/ml, in a weight ratio of 99:1 w/w to 4:1 w/w, preferably in a weight ratio as defined in item 1,
b) dissolving and mixing the at least one hyperbranched polymer and the at least one active pharmaceutical ingredient in an organic solvent, and
c) removing the solvent, wherein step b) or step c) further comprises combining the mixture with a hydrophilic or hydrophobic carrier, preferably a hydrophilic carrier, more preferably a crystalline carrier, even more preferably a carrier as defined above.

The process according to the invention is particularly advantageous in terms of process yield and better end solubility when the at least one active pharmaceutical ingredient and the hyperbranched polymer is dissolved in the solvent. In this case, and under proviso the weight ratio requirement is met, the active pharmaceutical ingredient is obtained in amorphous form, being in contact with the hyperbranched polymer over a large surface area and extensively interacting with the polymer. Preferably, a solid dispersion is obtained.

When preparing a powder or granulate, in step b) a hydrophilic or hydrophobic carrier, preferably hydrophilic carrier, more preferably a carrier as defined above, is added. When the carrier is in the form of particles, it is preferred that step b) is performed by depositing the hyperbranched polymer and active pharmaceutical ingredient on the surface of the carrier particles. The constituents get deposited on the carrier preferably by applying techniques like high shear mixing, spray drying or fluid bed granulation, or the like. When carrying out high shear mixing for example, the constituents are preferably still at least partly dissolved in the solvent when they reach the carrier and the solvent spreads over the carrier before being completely removed. On the other hand, the parameters selected for the spray drying technique can, in principle, cause the solvent to evaporate before the constituents hit the carrier particles. Such conditions are less preferred. These techniques usually comprise to remove at least some of the solvent. However, a further step for solvent removal might be necessary.

However, the step of depositing the at least one pharmaceutically active ingredient and the at least one hyperbranched polymer can also be performed by simply mixing the components in the organic solvent and removing the solvent, wherein the solvent removal is also preferably carried out under mixing.

It is further preferred to add another pharmaceutically active ingredient as described above during the inventive process. According to the invention, the additional pharmaceutically active ingredient can be added during the preparation of the solid dispersion of hyperbranched polymer, pharmaceutically active ingredient and carriers. However, the additional pharmaceutically active ingredient can also be added after the solid dispersion has been prepared. Furthermore, the additional pharmaceutically active ingredient can be added to the coating of a tablet or can be mixed with the powder or granulate containing the solid dispersion according to the invention.

It is additionally preferred to add further excipients during the process according to the invention. Suitable excipients are described herein.

When using a powder or granulate for the preparation of a pharmaceutical dosage form, e.g. a tablet or capsule, it is preferred to compress the powder or granulate into a tablet, or fill the powder or granulate into a capsule.

The preparation of a solid dosage form can be carried out by direct compression of the pharmaceutical composition, powder or granulate as described herein and optional further excipients obtained in process step c) into tablets. However, the pharmaceutical composition, powder or granulate as described herein and optional further excipients can also be firstly granulated and then compressed in a tablet press to obtain tablets. Double- or multilayer-mode can be used on the tablet press to prepare double- or multi-layered tablets. Also, a tablet press can be used to obtain mantle coating. Optionally, the tablet is further mantle coated or film coated. Film coating can be prepared by dissolving or dispersing suitable polymer for coating and if necessary plasticizers, stabilizers (antioxidants, acidic or basic agents, etc.) or the like in a vehicle and depositing the vehicle on the dosage form. The process can be done for example by spraying technique or dip coating.

Example of a suitable apparatus to be used is a coating pan. Mantle coating can be achieved by compacting the pharmaceutical formulation around the dosage form. Alternatively, the pharmaceutical composition according to the present invention, optionally being first transformed to the powder or the granulate, can be filled in the gelatine or hypromellose capsules. Also, various spheronization or extrusion techniques can be employed to prepare pellets. Pellets and capsules may be further coated.

The process of drying the pharmaceutical composition, powder, granulate or pharmaceutical dosage form further enhances the stability and handling properties. The drying process can be applied at any step in the preparation of the composition, powder, granulate, tablet, pellet, capsule, or the like.

In addition, the present invention refers to the use of a crystalline carrier for the preparation of a pharmaceutical composition comprising hyperbranched polymer and a pharmaceutically active ingredient. As discussed above, the use of crystalline carriers in the inventive pharmaceutical compositions provides advantageous properties.

The present invention also refers to the use of dendrimeric polyesteramide hyperbranched polymers for the preparation of a pharmaceutical composition comprising at least one active pharmaceutical ingredient, wherein the weight ratio of the hyperbranched polymer to the at least one active pharmaceutical ingredient is 99:1 w/w to 4:1 w/w. Further preferred dendrimeric polyesteramide hyperbranched polymers,APIs, weight ratios of hyperbranched polymer/API, etc., which can be used are described above.

Furthermore, the present invention relates to the use of a hydrophilic or hydrophobic carrier for the preparation of a pharmaceutical composition comprising an active pharmaceutical ingredient selected from the group consisting of phosphodiesterase inhibitors, non-thiazide sulphonamides, sulfonylurea derivatives, and hydroxy-1,4-naphthoquinone derivatives, preferably a non-thiazide sulphonamide, particularly preferred indapamide, in mixture with at least one hyperbranched polymer. Preferred hydrophilic carriers and preferred non-thiazide sulphonamides and further ingredients, weight ratios, etc. are described above. In addition, the present invention relates to the use of a hydrophilic carrier for the preparation of a pharmaceutical composition as defined above.

The present invention also relates to the use of an active pharmaceutical ingredient selected from the group consisting of phosphodiesterase inhibitors, non-thiazide sulphonamides, sulfonylurea derivatives, and hydroxy-1,4-naphthoquinone derivatives for the preparation of a pharmaceutical composition comprising at least one hyperbranched polymer, wherein the at least one pharmaceutically active ingredient exhibits a solubility in an aqueous media at pH 6.8 and 37° C of less than 1 mg/ml, and wherein the weight ratio of the at least one hyperbranched polymer to the at least one active pharmaceutical ingredient is 99:1 w/w to 4:1 w/w. Preferably, the pharmaceutical ingredient is selected from the group consisting of pimobendan, tadalfil, indapamide, glimepiride, and atovaquone. Preferred hyperbranched polymers, APIs, weight ratios of hyperbranched polymer/API, etc., which can be used are described above.

The following examples are merely illustrative of the present invention and they should not be considered as limiting the scope of the invention in any way, as these examples, modifications and other equivalents thereof will become apparent to those versed in the art in the light of the present disclosure, and the accompanying claims.

### Description of the figures

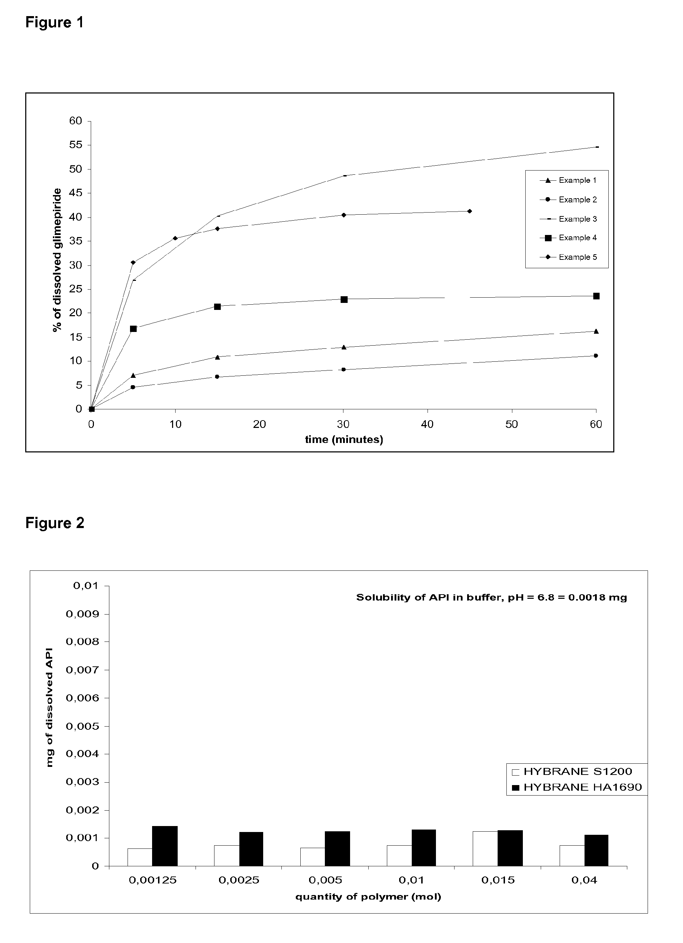

Fig. 1 shows the effect of surface active ingredients or inactive ingredients on the solubility of glimepiride.
Fig. 2 shows effects of hyperbranched polymers on the solubility of glimepiride.
Fig.3 shows a comparative dissolution study of glimepiride in phosphate buffer with pH 6.8 for samples containing Hybrane^{®} S1200.
Fig.4 shows a comparative dissolution study of glimepiride in phosphate buffer with pH 6.8 for samples containing Hybrane^{®} HA1690.

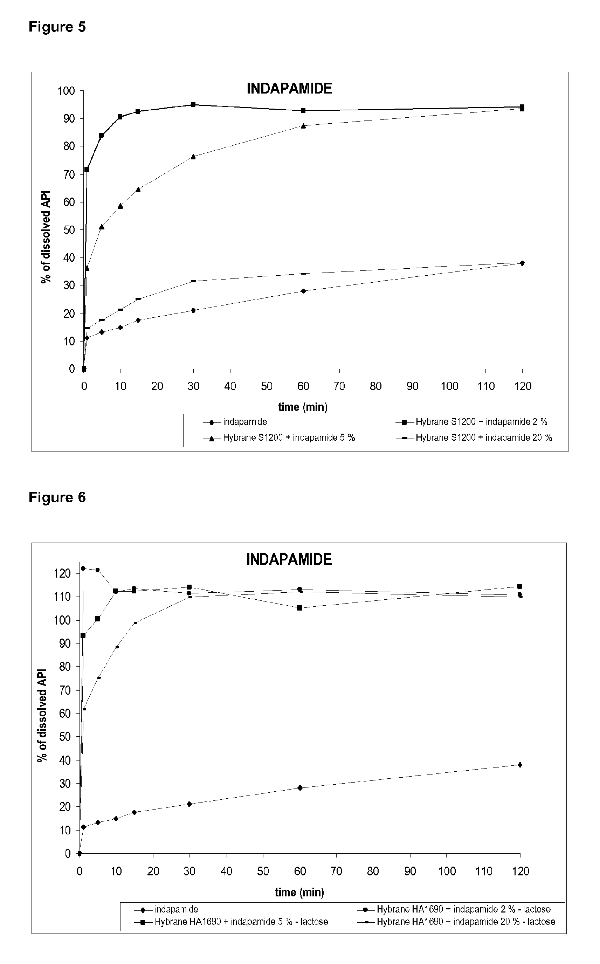

Fig.5 shows a comparative dissolution study of indapamide in phosphate buffer with pH 6.8 for samples containing Hybrane^{®} S1200.
Fig. 6 shows a comparative dissolution study of indapamide in phosphate buffer with pH 6.8 for samples containing Hybrane^{®} HA1690.
Fig. 7 shows a comparative dissolution study of pimobendan in phosphate buffer with pH 6.8 for samples containing Hybrane^{®} S1200.
Fig.8 shows hygroscopicity of the pharmaceutical compositions comprising only hyperbranched polymer and an active pharmaceutical ingredient in comparison with the compositions further comprising a carrier.

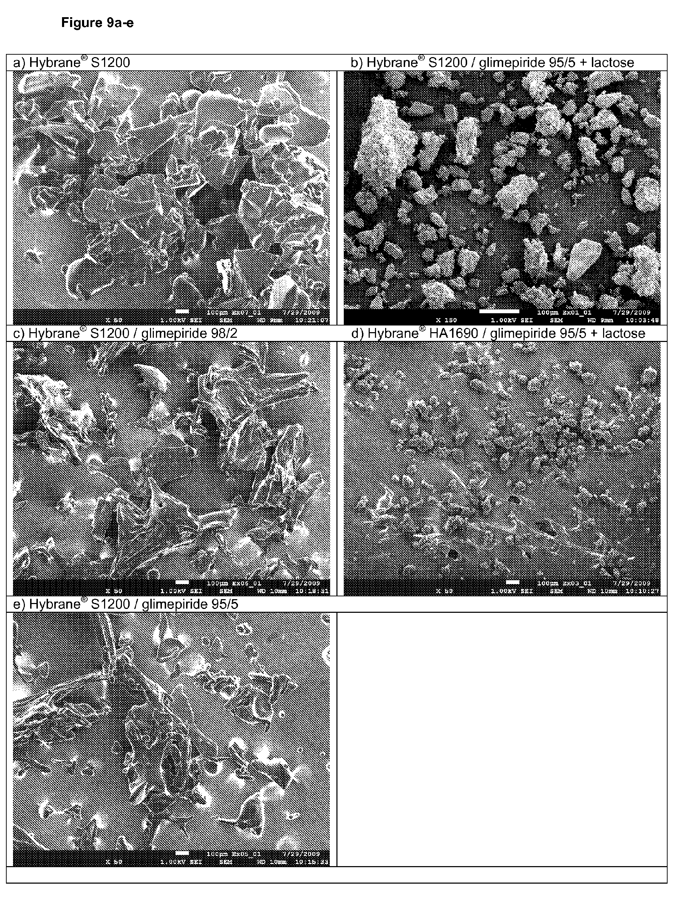

Fig. 9 shows scanning electron microscope images of pharmaceutical composition particles, their size, shape, and growth.
Fig. 10 shows hygroscopicity of a pharmaceutical dosage form comprising only hyperbranched polymer and an active pharmaceutical ingredient in comparison with the dosage form further comprising a carrier.
Fig. 11 shows hygroscopicity of a pharmaceutical composition comprising hyperbranched polymer and a pharmaceutically active ingredient alone, comprising a hyperbranched polymer, pharmaceutically active ingredient and a crystalline carrier (lactose 200 Mesh) and comprising a hyperbranched polymer, pharmaceutically active ingredient and an amorphous carrier (spray dried lactose), respectively.

### Examples

### Comparative examples 1 - 5 (see Fig. 1)

Comparative Example 1: Simple mixture of glimepiride and Texapon (sodium lauryl sulfate)-9 %w/w
Comparative Example 2: Simple mixture of glimepiride and Polysorbate - 9 % w/w
Comparative Example 3: Granulate comprising of milled glimepiride, Polysorbate and lactose (4/1/95 % w/w)
Comparative Example 4: Granulate comprising of unmilled glimepiride, Polysorbate and lactose (4/1/95 % w/w)
Comparative Example 5: Duetact 30/4 *
   * Glimepiride is currently marketed in a combination product with pioglitazone hydrochloride under the name Duetact (Takeda). Originator company has improved the low solubility by incorporation of a surfactant into a formulation (Polysorbate 80).

Because of the low water solubility and pH dependency of glimepiride, some pharmaceutically accepted surfactants and inactive ingredients were tested in order to increase the solubility of glimepiride. Results are presented in figure 1. It can be seen from the results on figure 1 that none of the used inactive ingredients gave satisfactory results in terms of significantly improved solubility. Approximately 50 % of the active ingredient (4 mg) is dissolved after 60 minutes of testing at following conditions: 37 °C in the USP Dissolution Tester, Apparatus II (Paddle method) using 900 ml of phosphate buffer (pH 6.8) as the dissolution medium and at a rotation rate of 75 rpm.

In the following experiments hyperbranched polyesteramides (Hybrane^{®}) obtained from DSM were used. Used were: Hybrane^{®} S1200 and Hybrane^{®} HA1690.

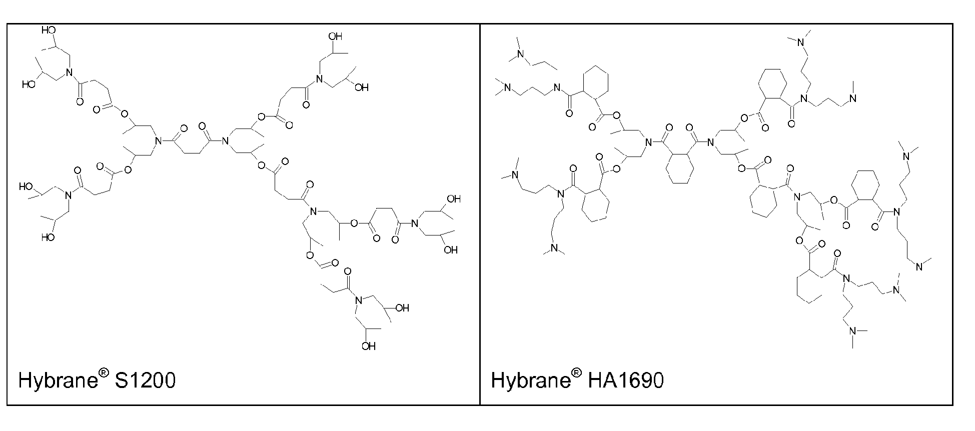

### Characteristics of the polymers:

| **Name** | **Colour** | **State** | **Solubility (water)** | **Solubility (ethanol)** | **End groups** |
|---|---|---|---|---|---|
| Hybrane^{®} S1200 | white | solid | soluble | soluble | hydroxyl |
| Hybrane^{®} HA1690 | Brown-yellow | Hygroscopic solid | soluble | soluble | Tertiary amine |

Hybrane^{®} HA 1690 represents a dendrimeric polyesteramide, wherein the structural units are hexa hydro phthalicacid **anhyhdride, diisopropanolamine and** N, N-bis-(3-dimethylaminopropyl)amine, and wherein the average molecular weight is 1.600. The provision of said polymers is described in the following publications:
1. Froehling P.: Development of DSM's Hybrane® hyperbranched polyesteramides. J Polym Sci A 1. 42. 2004. 3110 - 3115.
2. Froehling P., Brackman J.: Properties and applications of poly(propylene imine) dendrimers and poly(esteramide) hyperbranched polymers. Macromol Symp. 151. 2000. 581 - 589.
3. Dritsas G. S. et al.: Investigation of thermodynamic properties of hyperbranched poly (ester amide) by inverse gas chromatography. Journal of polymer science. Part B: Polymer physics. 2008. 46 : 2166-2172.

It has been unexpectedly found that the solubility of the API can be particularly increased if a dendrimeric polyesteramide having tertiary amine groups, preferably Hybrane^{®} HA 1690, is used as the hyperbranched polymer. The same was observed if Hybrane^{®} S1200 was used.

### Examples 6 - 13

Effect of hyperbranched polymer on the solubility of an active pharmaceutical ingredient when combined in a powder or granulate

Glimepiride was intimately mixed with the hyperbranched polymers as follows: First. a physical mixture was prepared and then glimepiride and the polymer were dissolved in the mutual solvent isopropyl alcohol at room temperature while stirring continuously (100 rpm) with magnetic stirrer for 1 hour. The solution prepared was transparent. Then the solvent was removed (so called solvent method). In cases where semi-solid or liquid product was obtained, granulation procedure was used. Polymer and the drug (i.e. API) were dissolved in a mutual solvent and then the solution was applied onto the crystalline lactose particles. The solution was applied in a fine spray onto the inert crystalline particles of lactose in the mortar and the forming mixture was mixed to ensure homogeneity. Technically, this can also be achieved for example with fluid bed granulation or in a high shear mixer. The moist mass was then dried in a vacuum oven. The temperature needed to assure evaporation of isopropyl alcohol was 40 °C. After 3 days the samples were removed form the vacuum oven and a solid end product was obtained which was then manually milled to powder and sieved through a sieve with 250 µm pores. The process was repeated for preparing compositions of various polymer/active ingredient weight ratios.

The composition of the samples is presented in the table bellow.

| **Ingredient** | **function** | **2 % w/w** | **5 % w/w** | **20 % w/w** |
|---|---|---|---|---|
| | | | | |
| Glimepiride | Active ingredient | 2 mg | 5 mg | 20 mg |
| Lactose | Inactive carrier | 400 mg | 400 mg | 400 mg |
| Hyperbranched polymer* | Solubilizing agent | 98 mg | 95 mg | 80 mg |

| | | | | |
|---|---|---|---|---|
| * Hyperbranched polymer: Hybrane^{®} S1200, Hybrane^{®} HA1690 | | | | |

Samples containing Hybrane^{®} S1200 (Fig. 3):

| Ingredients: | Glimepiride [mg] | Polymer [mg] | Lactose [mg] |
|---|---|---|---|
| Example 6 | 2 | 98 | / |
| Example 7 | 2 | 98 | 400 |
| Example 8 | 5 | 95 | / |
| Example 9 | 5 | 95 | 400 |
| Example 10 | 20 | 80 | 400 |

Samples containing Hybrane^{®} HA1690 (Fig. 4):

| Ingredients: | Glimepiride [mg] | Polymer [mg] | Lactose [mg] |
|---|---|---|---|
| Example 11 | 2 | 98 | 400 |
| Example 12 | 5 | 95 | 400 |
| Example 13 | 20 | 80 | 400 |

### In vitro dissolution studies:

Dissolution of glimepiride was assessed at 37 °C by the USP Dissolution Tester, Apparatus II (Paddle method) at a rotation rate of 75 rpm using 900 ml of phosphate buffer (pH 6.8) as dissolution medium. The tested samples were added directly to the buffer in the correct amount to achieve a final concentration of 4,4 µg / mL glimepiride (equals therapeutic dose dissolved in 900 mL). Aliquots, each of 2 ml, from the dissolution medium were withdrawn at time intervals of 5, 15, 30, 60 minutes. The samples were withdrawn through syringe and filtered through Millipore filter and analyzed for glimepiride content using HPLC method.

### HPLC assay determination:

The amount of dissolved glimepiride was estimated by reversed-phase HPLC (Waters Alliance) in a binary mode, with a photodiode array detector and a communication bus module. The analysis was performed at 230 nm with a C18, 150 x 4.6 mm, 3.5 µm column maintained at 30 °C (column oven) using a mobile phase A (phosphate buffer, pH = 2.5 : acetonitrile = 72 : 28) and mobile phase B (phosphate buffer, pH = 2.5 : acetonitrile = 30 : 70), delivered at a flow-rate of 1.5 mL/min and following gradient:

| time (min) | %A |
|---|---|
| 0 | 100 |
| 6 | 0 |
| 6,5 | 100 |
| 7,5 | 100 |

The retention time of the drug was approximately 4 min.

The effect of mixing of the drug with the hyperbranched polymers, which can be characterized as non-covalent association or complexation on the solubility of glimepiride in phosphate buffer solutions was investigated at 37 °C. It is evident from figures 3 and 4 that the solubility of glimepiride was significantly increased when using the specific ratio of hyperbranched polymer/pharmaceutically active ingredient according to the invention.

### Example 14

The samples of indapamide were prepared as in examples 6 to 13. Dissolution of indapamide was assessed at 37 °C by the USP Dissolution Tester, Apparatus II (Paddle method), using 900 ml of phosphate buffer (pH 6.8) as the dissolution medium and at a rotation rate of 75 rpm. The tested samples were added directly to buffer in an amount in order to achieve a final concentration of 15 µg / mL indapamide. Aliquots, each of 2 ml, from the dissolution medium were withdrawn at time intervals of 5, 15, 30, 60 minutes. The samples were withdrawn through syringe and filtered through Millipore filter in order to separate the undissolved sample and analyzed for indapamide using UV spectrofotometry at 287 nm.

It is vivid from figure 5 that the polyesteramide hyperbranched polymers remarkably enhance the solubility of the indapamide.

### Example 15

The samples of pimobendan were prepared as in examples 6 to 13. Dissolution of pimobendan was assessed at 37 °C by the USP Dissolution Tester, Apparatus II (Paddle method) at a rotation rate of 75 rpm, using 900 ml of phosphate buffer (pH 6.8) as the dissolution medium. The tested samples were added directly to the buffer in the correct amount to achieve a final concentration of 5.5 µg / mL pimobendan (equals therapeutic dose dissolved in 900 mL). Aliquots, each of 2 ml, from the dissolution medium were withdrawn at time intervals of 5, 15, 30, 60 minutes. The samples were withdrawn through syringe and filtered through Millipore filter in order to separate the undissolved sample and analyzed for pimobendan using HPLC method.

It has been found that polyesteramide Hybrane^{®} S1200 when admixed with pimobendan (2% and 5% of pharmaceutically active ingredient) significantly increases the solubility of the active ingredient.

### Example 16

The combination of an active ingredient and hyperbranched polymer was evaluated for the effect it has on the solubility of the following compounds: tadalafil, atovaquone, indapamide. The samples were prepared according to the process described in example 6 and exhibited an increase in the solubility of the respective active ingredient. The increase was 55-fold, 100-fold and 140-fold, respectively.

Maximum solubility in phosphate buffer,pH = 6.8 for samples containing 5 % w/w

| | Solubility without polymer (mg/mL) | Solubility in the presence of Hybrane S1200 (mg/mL) | Increase (fold) |
|---|---|---|---|
| Glimepiride | 0.001 | 0.01 | 10 |
| Pimobendan | 0.004 | 0.21 | 50 |
| Tadalafil | 0.002 | 0.11 | 55 |
| Atovaquone | 0.00003 | 0.003 | 100 |
| Indapamide | 0.005 | 0.7 | 140 |

### Example 17

Samples of the composition as presented in examples 9, 12, 6 and 8 were tested for hygroscopicity. Water intake was measured when exposed to relative humidity of 75 % at 25 °C. The conditions were preserved with the help of saturated solution of sodium chloride, kept at 25 °C. Relative weight gain was determined after 3 and 24 hours. The increase in weight in samples containing only hyperbranched polymer and active pharmaceutical ingredient was significantly higher in comparison to the increase in weight of samples containing hyperbranched polymer, active pharmaceutical ingredient and hydrophylic carrier lactose. Adsorbed/absorbed water in the samples containing only hyperbranched polymer and active pharmaceutical ingredient caused liquefaction of the samples, i.e. transformation from solid state to liquid. High hygroscopicity of the samples made the handling difficult. It was observed that the granulation procedure improved the handling significantly. Hygroscopicity decreased due to the incorporation of the carrier. After 24 hours at 75 % relative humidity only 3 % increase in weight was observed. The granulate remained in solid form and could be easily handled. The results are presented in figure 8. Furthermore, it was observed that hygroscopicity was mostly decreased if a crystalline carrier (e.g. lactose 200 Mesh) was used compared to an amorphous carrier (e.g. spray dried lactose). The results are presented in figure 11.

### Example 18

The samples as in example 17 were studied using scanning electron microscopy. The particle size, shape, aggregation, growth and liquefaction was evaluated. The samples comprising also lactose as the carrier were seen to remain in clear and distinct particulate form, without increase in size, change in shape and general liquefaction, whereas the samples comprising only the hyperbranched polymer and glimepiride as the active ingredient tend to built aggregates, to grow in size and change the shape. Thus, figure 9 shows that using a carrier material, e.g. lactose, stabilizes a pharmaceutical composition and allows maintaining the original properties of the pharmaceutical composition during storage

### Example 19

Samples as in example 17 that contained only the hyperbranched polymer and the active pharmaceutical ingredient (samples of the composition as presented in example 8) were compressed into approximately 100 mg tablets and the ones that contained also the hydrophilic carrier lactose (samples of the composition as presented in example 9) were compressed into approximately 500 mg tablets by using hydraulic press with force of 10000 N.

Tablets were then exposed to relative humidity of 75 % at 25 °C as in example 17. Relative weight gain was determined after 3 and 24 hours. The increase in weight in tablets containing only hyperbranched polymer and drug was significantly higher compared to the increase in weight of tablets containing also lactose. As seen before in powder samples, adsorbed/absorbed water in the samples containing only hyperbranched polymer and active pharmaceutical ingredient caused liquefaction of the tablets. The results are presented in figure 10.

### Example 20

Chemical stability of the 500 mg tablets that contained hyperbranched polymer, active pharmaceutical ingredient and lactose, as well as chemical stability of the granulate was determined. Samples were exposed to stress condition of elevated temperature (50 °C) for 3 days. Higher temperature was not applied due to sensitivity of inactive ingredients to the elevated temperature.

When mixing different ingredients in a pharmaceutical formulation there exists the possibility that components interact with each other. In addition, each component may have different degradation characteristics. This can subsequently lead to problems arising from the chemical stability of the active pharmaceutical ingredient in the dosage form.

The HPLC analysis were performed with a Waters system equipped with a C18, 5 µm, 150 X 4.6 mm column which was maintained in a column oven at 30 °C. The mobile phase consisted of a mixture of a phosphate buffer, pH = 2.5 and acetonitrile (50: 50 V/V). The flow rate was 1 mL/min, and the detection wavelength was 228 nm. All impurities eluted within 15 minutes represent the total amount of glimepiride impurities.

Comparative stability study of the samples is presented in the table below:

| Example no | Initial (%) | Sum of impurities after 3 days at 50 °C (%) | Sum of impurities after 24 h at 75 % RH (%) |
|---|---|---|---|
| **Granulate -** Glimepiride/Hybrane/lactose | 0.52 | 0.63 | / |
| **Tablets -** Glimepiride/Hybrane/lactose | / | 0.56 | 0.51 |

It can be seen from the results above that no significant degradation occurred after 3 days at elevated temperature of 50 °C. The glimepiride as active pharmaceutical ingredient was stable and compatible with the chosen constituents.

## Claims

1. A pharmaceutical composition comprising:
a) at least one hyperbranched polymer; and
b) at least one pharmaceutically active ingredient exhibiting solubility in an aqueous media at pH 6.8 and 37° C of less than 1 mg/ml, and
wherein the weight ratio of the at least one hyperbranched polymer to the at least one active pharmaceutical ingredient is 99:1 w/w to 11:1 w/w, or wherein the weight ratio is 99:1 w/w to 4:1 w/w if the active pharmaceutical ingredient is selected from the group consisting of phosphodiesterase inhibitors, non-thiazide sulphonamides, sulfonylurea derivatives, and hydroxy-1,4-naphthoquinone derivatives, or wherein the weight ratio is 99:1 w/w to 4:1 w/w if a dendrimeric polyesteramide is used as the hyperbranched polymer.

2. The pharmaceutical composition according to claim 1, wherein the hyperbranched polymer and the at least one pharmaceutical ingredient form a solid dispersion which optionally further comprises at least one water-dispersible or water-soluble pharmaceutically acceptable carrier.

3. The pharmaceutical composition according to claim 1 or 2, wherein the at least one pharmaceutically active ingredient is selected from the group consisting of glimepiride, gliclazide, glipizide, glibenclamide, pimobendan, tadalafil, atovaquone and indapamide, preferably glimepiride, pimobendan, tadalafil, atovaquone and indapamide, more preferably the pharmaceutically active ingredient is glimepiride.

4. Powder or granulate comprising
a) at least one hyperbranched polymer,
b) at least one pharmaceutically active ingredient exhibiting a solubility in an aqueous media at pH 6.8 and 37° C of less than 1 mg/ml,
wherein the weight ratio of the at least one hyperbranched polymer to the at least one active pharmaceutical ingredient is 99:1 w/w to 4:1 w/w, and
c) a hydrophilic or hydrophobic carrier.

5. Pharmaceutical dosage form comprising the pharmaceutical composition according to claim 1 or 2 or the powder or granulate according to claim 4, wherein preferably the pharmaceutical dosage form is a solid dosage form.

6. A pharmaceutical composition, powder, granulate or pharmaceutical dosage form according to any of the preceding claims, wherein the pharmaceutical composition, powder, granulate or pharmaceutical dosage form does not contain a surfactant.

7. A process for preparing a pharmaceutical composition comprising the steps of:
a) providing at least one hyperbranched polymer and at least one active pharmaceutical ingredient exhibiting a solubility in an aqueous medium at pH 6.8 and 37° C of less than 1 mg/ml, in a weight ratio of 99:1 w/w to 11:1 w/w, or wherein the weight ratio is 99:1 w/w to 4:1 w/w if the active pharmaceutical ingredient is selected from the group consisting of phosphodiesterase inhibitors, non-thiazide sulphonamides, sulfonylurea derivatives, and hydroxy-1,4-naphthoquinone derivatives, or wherein the weight ratio is 99:1 w/w to 4:1 w/w if a dendrimeric polyesteramide, preferably having tertiary amine groups, is used as the hyperbranched polymer,
b) forming a mixture of the at least one hyperbranched polymer, the at least one active pharmaceutical ingredient and an organic solvent, and
c) removing the organic solvent.

8. A process for preparing a powder or granulate comprising the steps of:
a) providing at least one hyperbranched polymer and at least one active pharmaceutical ingredient exhibiting a solubility in an aqueous medium at pH 6.8 and 37° C of less than 1 mg/ml, in a weight ratio of 99:1 w/w to 4:1 w/w,
b) forming a mixture of the at least one hyperbranched polymer, the at least one active pharmaceutical ingredient and an organic solvent, and
c) removing the solvent and preparing a powder or granulate, wherein step b) or step c) further comprises combining the mixture with a hydrophilic or hydrophobic carrier, preferably a crystalline carrier.

9. The process according to claims 7 or 8, wherein the organic solvent is selected from the group consisting of C₂-C₄ alkanols, such as ethanol, n-propanol, isopropanol, n-butanol, isobutanol; aliphatic or alicyclic ether, such as diethyl ether, diisopropyl ether, methyl tert-butyl ether, dioxane, tetrahydrofurane; ketone, such as acetone, methyl ethyl ketone; dimethylsulphoxide and dimethylformamide; or mixtures thereof.

10. The process according to claims 7 to 9, wherein step c) is performed by using high shear mixing, spray drying or fluid bed granulation.

11. Use of a crystalline carrier for the preparation of a pharmaceutical composition comprising hyperbranched polymer and a pharmaceutically active ingredient.

12. Use of dendrimeric polyesteramide hyperbranched polymers for the preparation of a pharmaceutical composition comprising at least one active pharmaceutical ingredient, wherein the weight ratio of the hyperbranched polymer to the at least one active pharmaceutical ingredient is 99:1 w/w to 4:1 w/w.

13. Use of a hydrophilic or hydrophobic carrier for the preparation of a pharmaceutical composition comprising a pharmaceutically active ingredient selected from the group consisting of phosphodiesterase inhibitors, non-thiazide sulphonamides, sulfonylurea derivatives, and hydroxy-1,4-naphthoquinone derivatives, in mixture with at least one hyperbranched polymer.

14. Use of an active pharmaceutical ingredient selected from the group consisting of phosphodiesterase inhibitors, non-thiazide sulphonamides, sulfonylurea derivatives, and hydroxy-1,4-naphthoquinone derivatives for the preparation of a pharmaceutical composition comprising at least one hyperbranched polymer, wherein the at least one pharmaceutically active ingredient exhibits a solubility in an aqueous media at pH 6.8 and 37° C of less than 1 mg/ml, and wherein the weight ratio of the at least one hyperbranched polymer to the at least one active pharmaceutical ingredient is 99:1 w/w to 4:1 w/w.

15. Use according to claim 14, wherein the pharmaceutical ingredient is selected from the group consisting of pimobendan, tadalfil, indapamide, glimepiride, and atovaquone.
